# EUROPEAN PATENT APPLICATION

(11) **EP 1 479 778 A1**
(43) Date of publication of application: **24.11.2004**
(21) Application number: 03253140.2
(22) Date of filing: 20.05.2003
(51) Int. Cl.: C12Q 1/00, C12Q 1/60, G01N 27/403, G01N 33/49, G01N 33/487

(54) **Electrochemical sensor with sample pretreatment**

(71) Applicant: Apex Biotechnology Corporation, Hsinchu, Tawain (CN)
(72) Inventor: Shen, Joseph C.L., Hsinchu, Roc (TW); Liu, I-Chung, Hsinchu, Roc (TW); Lin, Yueh-Hui, Hsinchu, Roc (TW); Huang, Ying-Che, Hsinchu, Roc (TW); Shen, Benjamin Cheng-Yu, Hsinchu, Roc (TW); Chin, Li-Te, Hsinchu, Roc (TW); Shen, Thomas Y.S., Hsinchu, Roc (TW)
(74) Representative: Nicholls, Michael John

(57) **Abstract**

The present invention discloses an electrochemical sensor with sample pretreatment function, which comprises:
an insulating substrate,
an electrode system disposed on the substrate, which comprises at least a working electrode and a reference electrode,
an electrically insulating layer partially covering the electrode system, so an exposed area of the electrode system not covered by the electrically insulting layer forms an electrochemical electrode reaction region that accommodates sample solution and an air vent,
a reaction layer disposed in the electrochemical electrode reaction region and comprising at least an oxidoreductase, an electron mediator, and at least a carrier, and
a pretreatment membrane layer disposed on the reaction layer of the electrode reaction region, which is capable of physically or chemically treating the sample solution, wherein the sample solution is pretreated through the pretreatment membrane layer of the sensor and enters the electrochemical electrode reaction region covered with the reaction layer to undergo reaction with the oxidoreductase and the electron mediator.

## Description

### BACKGROUND OF THE INVENTION

### 1. Field of the Invention

The present invention relates to a kind of electrochemical sensor with sample pretreatment function. In particular, said electrochemical sensor can pretreat a blood sample and measure the level or unit activity of a target to be tested in the blood sample by undergoing an electrochemical reaction with an oxidoreductase and an electron mediator.

### 2. Description Of Related Arts

Biochemical tests are done predominantly using optical instruments, which often require large volume of blood sample (about 5 milliliters), while using minute amount (10 to 100 microliters) for analysis, which results in large amount of medical waste and produces negative impact on the patients mentally. In addition, blood cells sometimes need to be removed from the blood sample by centrifuging so the assay process may be free from the interference of blood cells or hemolysis. For example, in assaying the unit activity of triglyceride, cholesterol level, or enzyme using automated optical instrument, blood sample still needs to be pretreated to rid the interference of red blood cells via the surfactant or hypotonic solution contained in the reagent so as to obtain tens of microliters of blood plasma or serum for subsequent analysis (e.g. remove the absorbance interference of hemoglobin in red blood cell).

A few patents have been granted to bioassay techniques using electrochemical approach, such as US Patent No. 5,120,420 and 5,762,770 as well as ROC Patent No, 124332, Methods disclosed in those patents allow bioassay of whole blood samples. But in some biochemical analyses using electrochemical techniques, such as assay of cholesterol, ALT or AST, the reagent still contains surfactant or hypotonic solution, meaning the blood sample assayed must be pretreated plasma or serum that is free of blood cell interference for proceeding enzymatic oxidoreductase and electrochemical reactions without deviation. Some electrochemical sensor inventions attempt to arrange a pretreatment element to remove blood cell interference during the assay of whole blood sample. But the above sensors need more materials or steps to complete the assay. For instance, US Patent No, 6,033,866 discloses the use of Ag/AgCl electrode coupled with two electron mediators. US Patent No. 6,436,255 B2 discloses the use of cover member for sampling by siphonic action.

Therefore, a new electrochemical sensor having advantages of being manufactured easily, having analysis results of high precision, and analyzing target to be tested directly in whole blood samples, is desired.

### SUMMARY OF THE INVENTION

An objective of the present invention is to provide an electrochemical sensor with the function of sample pretreatment.

Particularly, the electrochemical sensor of the invention comprises:
an insulating substrate;
an electrode system disposed on the surface of substrate comprising at least a working electrode and a reference electrode;
an electrically insulating layer partially covering the electrode system to form an electrochemical electrode reaction region that can accommodate sample solution as well as an air vent in an exposed area of the electrode system not covered by the electrically insulting layer;
a reaction layer disposed in the electrode reaction region comprising at least an oxidoreductase, an electron mediator, and at least a carrier; and
a pretreatment membrane layer disposed on the reaction layer in the electrode reaction region, which is capable of physically or chemically treating the sample solution, wherein the sample solution is pretreated through the pretreatment membrane layer of the sensor and enters the electrode reaction region covered with the reaction layer to undergo reaction with the oxidoreductase and the electron mediator.

### BRIEF DESCRIPTION OF THE DRAWINGS

FIG. 1A is an exploded view and a constitutional diagram of an electrochemical sensor according to the present invention.
FIG. 1B is a longitudinal sectional view of the sensor in FIG. 1A.
FIG. 1C is a horizontal sectional view of the sensor in FIG. 1A.
FIG. 2 is a relational graph of cholesterol level in whole blood sample versus current intensity as assayed by a sensor in the Example 1 of the present invention.
FIG. 3A is an exploded view and a constitutional diagram of another electrochemical sensor in accordance with the present invcntion,
FIG. 3B is a longitudinal sectional view of the sensor in FIG. 3A.
FIG. 3C is a horizontal sectional view of the sensor in FIG. 3A.
FIG. 4 is a relational graph of unit activity of ALT in whole blood sample versus current intensity as assayed by a sensor in Example 2 of the present invention.

### DETAILED DESCRIPTION OF THE INVENTION

According to the present invention, the blood samples are pretreated with the electrochemical sensor to eliminate the reactions or effects of detecting reagents with blood components (e.g. decomposition of red blood cells) that will interfere with the assay, and then the pretreated sample can proceed the enzymatic oxidoreductions and the electrochemical reactions. From the results of those reactions, the level or unit activity of the target to be tested may be easily obtained. Moreover, the preparation of the sensor according to the present invention involves only simple processed materials and steps.

The present invention provides an electrochemical sensor having the function of sample pretreatment, comprising:
an insulating substrate;
an electrode system disposed on the surface of substrate comprising at least a working electrode and a reference electrode;
an electrically insulating layer partially covering the electrode system to form an electrochemical electrode reaction region that can accommodate sample solution as well as an air vent in an exposed area of the electrode system not covered by the electrically insulting layer;
a reaction layer disposed in the electrode reaction region comprising at least an oxidoreductase, an electron mediator, and at least a carrier; and
a pretreatment membrane layer disposed on the reaction layer of electrode reaction region, which is capable of physically or chemically treating the sample solution, wherein the sample solution is pretreated through the pretreatment membrane layer of the sensor and enters the electrode reaction region covered with a reaction layer to undergo reaction with the oxidoreductase and the electron mediator.

The present invention also provides an electrochemical sensor having the function of sample pretreatment, comprising:
an insulating substrate;
an electrode system disposed on the surface of substrate comprising at least a working electrode and a reference electrode;
an electrically insulating layer partially covering the electrode system to form an electrochemical electrode reaction region that can accommodate sample solution in an exposed area of the electrode system not covered by the electrically insulting layer;
a reaction layer disposed in the electrode reaction region comprising at least an oxidoreductase, an electron mediator, and at least a carrier;
a hydrophilic mesh layer overlaying the electrode reaction region; and
a pretreatment membrane layer disposed on the hydrophilic mesh layer, which is capable of physically or chemically treating the sample solution, wherein the sample solution is pretreated through the pretreatment membrane layer of the sensor and enters the electrode reaction region covered with a reaction layer to undergo reaction with the oxidoreductase and the electron mediator.

As used herein, the "insulating substrate" refers to a thin laminate with flat straight surface and the property of electrical insulation, which is preferably selected from the group consisting of PVC plate, FR-4, polyester, phenolic resin sheet, PET plate, PC plate, PP plate, PE plate, PA plate, PS plate, glass plate, and CEM-1. According to the constitution of one embodiment of the present invention, said insulating substrate is a PC or PVC plate.

The "electrode system" as used herein refers to a system comprising at least two metallic electrodes separated from each other and forming respectively a working electrode and a reference electrode. According to the constitution of one embodiment of the present invention, the electrode system is partially covered by an electrically insulating layer, while one end of the exposed portion of the electrode system forms a working electrode and a reference electrode respectively and connect with the electrode reaction region, the other end of the exposed portion forms connections of the working electrode and the reference electrode, which may be connected with the sensing device generating the electrical effect when the sample undergoes electrochemical reaction. Preferably said electrode system can be composed of conductive paste materials suitable for screen printing, including but not limited to carbon paste, gold paste, silver paste, carbon-silver blended paste, volatile graphite, copper paste or their combinations (e.g. screen printing the silver paste and then the carbon paste), or any other conductive paste materials that are suitable for screen printing and can be dried under 80°C.

The "electrically insulating layer" as used herein refers to a thin layer made of materials with the property of electrical insulation, which preferably is selected from electrically insulating paste materials suitable for screen printing or electrically insulating tape materials, such as heat-dried or UV-dried insulating ceramic paint, or PVC or PET insulating tapes. Preferably said electrically insulating layer has a thickness of 0.1 to 0.25 millimeters. According to the constitution of one embodiment of the present invention, said electrically insulating layer partially covers the electrode system disposed on the substrate that the exposed region of the electrode system forms an electrochemical electrode reaction region that can accommodate the sample solution as well as an air vent.

The "reaction layer" as used herein refers to a thin layer of a formulation, which comprises at least an oxidoreductase, an electron mediator, and at least a carrier, disposed on the aforesaid electrode reaction region. Preferably, said formulation may be dried under 80°C after it is coated or added dropwise onto said electrode reaction region.

The "oxidoreductase" as used herein refers to an enzyme that can undergo oxidation and reduction with the target to be tested in the blood sample. The types of enzyme vary with different targets. For example, glucose oxidase and glucose dehydrogenase are used for glucose assay; xanthine oxidase is used for xanthine assay; glutamate oxidase is used for glutamate assay; lactate oxidase is selected to measure the level of lactate; creatinine oxidase is selected to measure the level of creatinine; β-dehydrogenase is used for the assay of β-hydroxybutyrate; cholesterol esterase and cholesterol oxidase are used for cholesterol assay; and pyruvate oxidase is selected to measure the activity of liver enzymes, including SOOT (or AST) or SGPT (or ALT).

The "electron mediator" as used herein refers to a substance that can itself be reduced from the oxidized state to the reduced state after reacting with a substance generated from the aforementioned oxidoreductase. When the electron mediator changes into the reduced state, an external voltage can be applied to the electrode strip to prompt the electron mediator returned to the oxidized state from the reduced. At this time, the variations of potential, resistance or current due to the chemical reaction can be transmitted from the working electrode and the reference electrode to the connections at the other ends of the electrode system. When said connections are connected to a signal receiving device, the device can receive the variations of potential, resistance or current due to the chemical reaction and convert the received signals into the level of the target to be tested through a display device. According to one embodiment of the present invention, said electron mediator may be potassium ferricyanide, whose content preferably ranges from 0.1 to 1.0 microgram (µg),

The "carrier" depicted herein means a substance that supports the dried oxidoreductase and electron mediator to adhere to the insulating substrate or a substance that can protect oxidoreductase. Preferably said carrier is, but not limited to a surfactant, albumin, serum protein, cellulose or cellulose derivative, or a mixture of those substances. Preferably the surfactant is Triton X-100, polyethylene glycol, monododecylether, sodium cholate or other water-soluble surfactants, According to one embodiment of the present invention, said carrier comprises surfactant (Triton X-100) and bovine serum albumin.

The "pretreatment membrane layer" depicted herein means a thin layer that can treat the blood sample solution physically (e.g. filtration or adsorption) or chemically (e.g. enzymatic catalyzation or co-valent bonding) to remove interferants in the sample solution and let the treated sample solution enter the electrode reaction region. Preferably said pretreatment membrane layer comprises at least one porous or cellulose material film with pore size in the range from 0.5 ~ 3 µm. According to one embodiment of the present invention, said pretreatment membrane layer comprises a layer of Hemasep V or BTS 100 (made by Pall Specialty Materials).

The "hydrophilic mesh layer" depicted herein means a thin layer containing a meshed membrane that can guide the fluid sample to permeate from one side of the mesh to the other side, which also vents air. Preferably said hydrophilic mesh layer contains a meshed membrane having 50-250/cm² mesh size.

The present invention is further described in detail by the following examples, which are only used to exemplify the present invention but not to limit the scope of the present invention. The modifications and changes achieved easily by persons skilled in the arts are included in the scope of the patent invention.

### Example 1

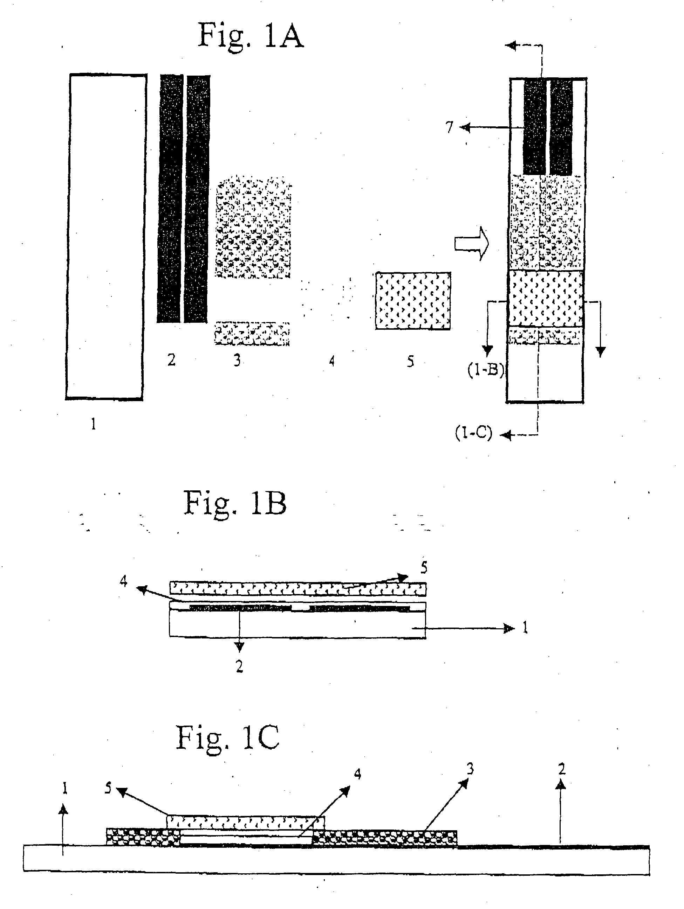

In this embodiment, a carbon paste electrode system was screen printed and an electrically insulating layer was formed on a PC-based insulating substrate in sequence, so as to form an electrochemical electrode reaction region and an air vent. Subsequently, a reaction fluid or slurry containing cholesterol esterase (15U), cholesterol oxidase (3U), potassium ferricyanide (0.5 µg), Triton X-100 (0.05 µg) and bovine serum albumin (BSA, 0.01 µg) was coated on the electrode reaction region and dried to form a reaction layer, and further, a Hemasep V membrane (made by Pall Specialty Materials) was overlaid on the reaction layer and the electrically insulating layer and used as a pretreatment membrane for physical removal of red blood cells from the blood sample. An electrochemical sensor formed by the above-mentioned methods was shown in FIG. 1.

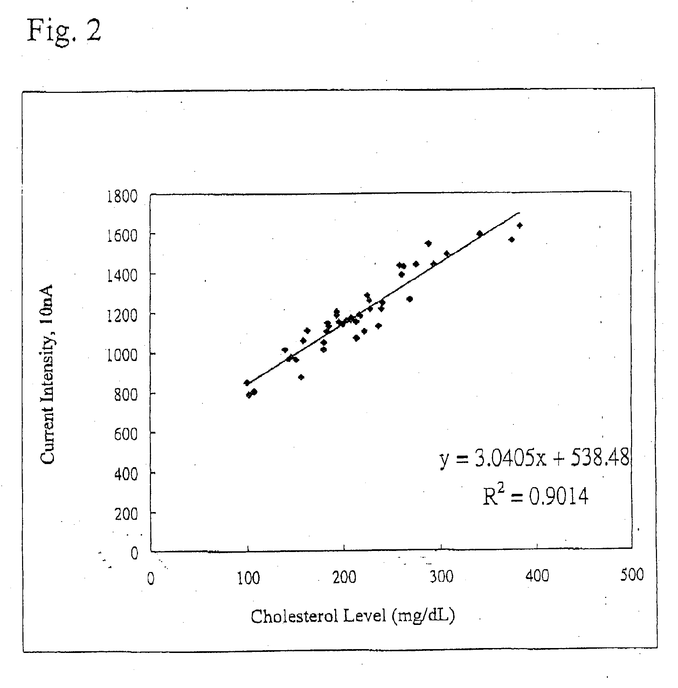

Then, the whole blood sample was introduced onto the pretreatment membrane. The pretreated blood plasma obtained through the pretreatment membrane entered the electrode reaction region, contacted with the formulation of the reaction layer, and underwent the reaction. An external electric field (300mV) is applied by CV50W for proceeding the electrochemical reaction, which resulted in the intensity of current signals. The current signals were analyzed and compared with other whole blood samples by utilizing an biochemical optical analysis procedure (a cholesterol reagent used was Product No. C-101-1 from Unison Biotech Co., Ltd.; a spectrophotometer used was Metertek Spectrophotometer SP8001). The analytical results as shown in FIG. 2 indicate that the electrochemical sensor having sample pretreatment function in accordance with the present invention is effective in measuring the level of cholesterol in blood.

### Example 2

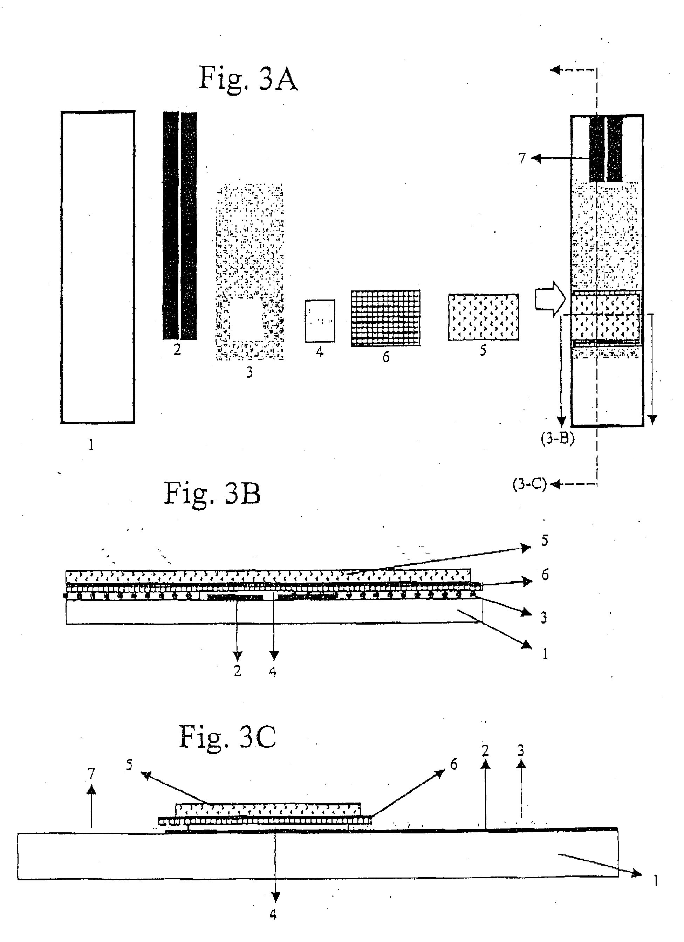

In this example, a silver/carbon paste electrode system was screen printed and an electrically insulating layer was formed on a PVC-based insulating substrate in sequence so as to form an electrode reaction region and an air vent. Subsequently, a reaction fluid or slurry containing pyruvate oxidase (0.06U), potassium ferricyanide (120 µg), Triton X-100 (0.006 µg) and bovine serum albumin (BSA, 0.03 µg) was coated on the electrode reaction region and dried to form a reaction layer. Further, a hydrophilic mesh layer (130 mesh size) overlaid the reaction layer and the insulating layer, on which a Hemasep V membrane (made by Pall Specialty Materials) was formed on the hydrophilic mesh layer and used as a pretreatment membrane for physical removal of red blood cells from the blood samples. An electrochemical sensor formed according to the above-mentioned methods was shown in FIG. 3.

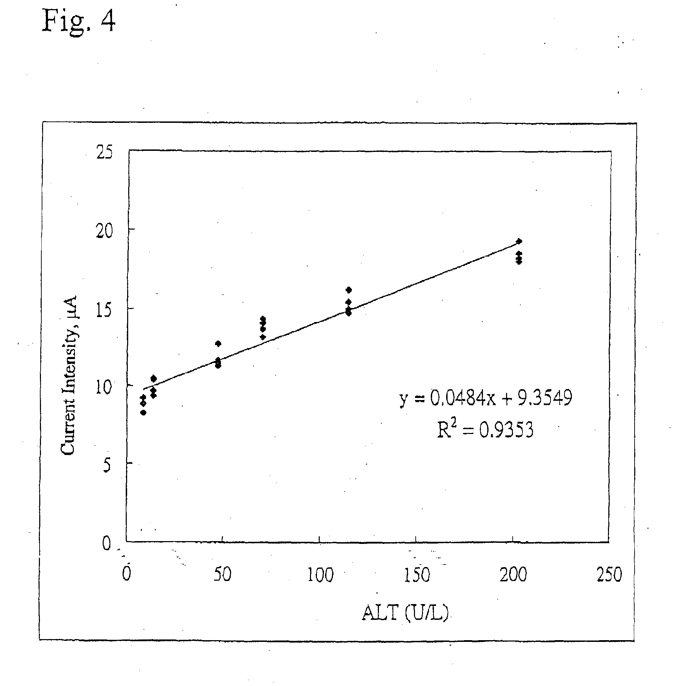

Next, alanine and α-keto-glutarate were added into a whole blood sample, which had an end concentration of 500mM and 150mM respectively. This whole blood sample was introduced onto the pretreatment membrane. The pretreated plasma obtained through the pretreatment membrane entered the electrode reaction region, contacted with the formulation of the reaction layer, and underwent reaction. An external electric field (400mV) is applied by CV50W for proceeding the electrochemical reaction, which resulted in the intensity of current signals. The current signals obtained thereof were analyzed and compared with other whole blood samples by utilizing an biochemical optical analysis procedure (an ALT reagent used was Product No. DG159-K from SIGMA; a spectrophotometer used was Metertek Spectrophotometer SP8001). The analytical results as shown in FIG. 4 indicate that the electrochemical sensor having sample pretreatment function in accordance with the present invention is effective in measuring the activity of ALT in blood.

## Claims

1. An electrochemical sensor having the function of sample pretreatment, comprising:
an insulating substrate;
an electrode system disposed on the surface of the substrate comprising at least a working electrode and a reference electrode;
an electrically insulating layer partially covering the electrode system to form an electrochemical electrode reaction region that can accommodate sample solution as well as an air vent in an exposed area of the electrode system not covered by the electrically insulting layer;
a reaction layer disposed in the electrode reaction region comprising at least an oxidoreductase, an electron mediator, and at least a carrier; and
a pretreatment membrane layer disposed on the reaction layer of the electrode reaction region, which is capable of physically or chemically treating the sample solution,
wherein after said sample solution is pretreated through the pretreatment membrane layer of the sensor, the pretreated sample solution enters the electrode reaction region covered with the reaction layer to undergo reaction with the . oxidoreductase and the electron mediator.

2. The electrochemical sensor according to Claim 1, wherein said electrically insulating layer has a thickness of 0.1 to 0.25 millimeter (mm).

3. The electrochemical sensor according to Claim 1 or 2, wherein said oxidoreductase is selected for the group consisting of cholesterol esterase, cholesterol oxidase, and mixtures thereof.

4. The electrochemical sensor according to Claim 1, 2 or 3, wherein said oxidoreductase is pyruvate oxidase.

5. The electrochemical sensor according to any one of Claims 1 to 4, wherein said electron mediator is potassium ferricyanide,

6. The electrochemical sensor according to any one of Claims 1 to 5, wherein said carrier comprises surfactant (Triton X-100) and bovine serum albumin.

7. The electrochemical sensor according to any one of Claims 1 to 6, wherein said physical treatment comprises filtration or adsorption.

8. The electrochemical sensor according to any one of Claims 1 to 7, wherein said chemical treatment comprises enzymatic catalyzation or co-valent bonding.

9. The electrochemical sensor according to any one of Claims 1 to 8, wherein said pretreatment membrane layer comprises at least one porous or cellulose material film with pore size in the range from 0.5 to 3 micrometers (µm).

10. An electrochemical sensor having the function of sample pretreatment, comprising:
an insulating substrate;
an electrode system disposed on the surface of substrate comprising at least a working electrode and a reference electrode;
an electrically insulating layer partially covering the electrode system to form an electrochemical electrode reaction region that can accommodate sample solution in an exposed area of the electrode system not covered by the electrically insulting layer;
a reaction layer disposed in the electrode reaction region comprising at least an oxidoreductase, an electron mediator, and at least a carrier;
a hydrophilic mesh layer overlaying the electrode reaction region; and
a pretreatment membrane layer disposed on the hydrophilic mesh layer, which is capable of physically or chemically treating the sample solution,
wherein after said sample solution is pretreated through the pretreatment membrane layer of the sensor, the pretreated sample solution enters the electrode reaction region covered with the reaction layer to undergo reaction with the oxidoreductase and the electron mediator.

11. The electrochemical sensor according to Claim 10, wherein said electrically insulating layer has a thickness of 0.1 to 0.25 millimeter (mm).

12. The electrochemical sensor according to Claim 10 or 11, wherein said oxidoreductase is selected from the group consisting of cholesterol esterase, cholesterol oxidase, and mixture thereof.

13. The electrochemical sensor according to Claim 10, 11 or 12, wherein said oxidoreductase is pyruvate oxidase.

14. The electrochemical sensor according to any one of Claims 10 to 13, wherein said electron mediator is potassium ferricyanide.

15. The electrochemical sensor according to any one of Claims 10 to 14, wherein said carrier comprises surfactant (Triton X-100) and bovine serum albumin.

16. The electrochemical sensor according to any one of Claims 10 to 15, wherein said physical treatment comprises filtration or adsorption.

17. The electrochemical sensor according to any one of Claims 10 to 16, wherein said chemical treatment is enzymatic catalyzation.

18. The electrochemical sensor according to any one of Claims 10 to 17, wherein said pretreatment membrane layer comprises at least one porous or cellulose material film with pore size in the range from 0.5 to 3 micrometers (µm).

19. The electrochemical sensor according to any one of Claims 1 to 18, wherein said hydrophilic mesh layer contains a meshed membrane having 50 to 250/cm² mesh size.
